# EUROPEAN PATENT APPLICATION

(11) **EP 4 670 502 A1**
(43) Date of publication of application: **31.12.2025**
(21) Application number: 24760612.2
(22) Date of filing: 23.02.2024
(51) Int. Cl.: A01N 1/02, C12N 5/0775

(54) **FORMULATION FOR CELL CRYOPRESERVATION**

(30) Priority: 24.02.2023 KR 20230025103
(71) Applicant: Encell Co., Ltd., Gangnam-gu, Seoul 06072 (KR)
(72) Inventor: JEON, Hong Bae, Seoul 06072 (KR); PARK, Sang Eon, Seoul 06072 (KR)
(74) Representative: Mewburn Ellis LLP
(86) International application number: PCT/KR2024/002374
(87) International publication number: WO 2024/177423

(57) **Abstract**

The present invention relates to a formulation for cell cryopreservation that can significantly improve the cell survival rate of cryopreserved cells, preferably stem cells. According to the present invention, the cell survival rate can be enhanced during the thawing process after freezing, thereby preventing the reduction in therapeutic efficacy that may occur during the storage and transportation of cell therapy products.

## Description

### [Technical Field]

The present invention relates to a cryopreservation formulation for cells, preferably stem cells, and more specifically, to a cryopreservation formulation of stem cells that contains glutathione as an active ingredient and can significantly improve the survival rate of cells such as cryopreserved stem cells.

### [Background Art]

Cryopreservation is commonly used to store cells, including stem cells. Since cells have different physiological characteristics depending on the origin thereof, tissue of origin, and internal microenvironment, appropriate cryopreservation methods must be applied. Stem cells, in particular, require optimized cryopreservation conditions to maintain the unique, specific division and differentiation capacities thereof. Since stem cells may be used as cell therapy products immediately after storage, the use of low-toxicity cryoprotective solutions is essential. Developing effective techniques for stem cell cryopreservation is also a critical step toward establishing stem cell banks. Cryopreservation is a method of preserving samples while freezing at ultra-low temperatures, typically -80°C to - 196°C, to maintain cellular function, enabling long-distance transport and long-term storage.

Cryoprotective agents reduce physical and chemical cell damage during freezing and thawing, thereby improving cell viability after thawing. Typically, a cryoprotective agent (CPA) consisting of 10% (v/v) dimethyl sulfoxide (DMSO) and 90% (v/v) fetal bovine serum (FBS) is used for cell cryopreservation. This combination has been widely used for various types of stem cells. However, the use of DMSO and FBS has many drawbacks. For example, serum (e.g., FBS) in cryoprotective agents may result in infections or immune responses (Kocaoemer et al., Stem Cells., 25:1270-1278, 2007). In particular, the serum used in cryoprotective agents are a mixture of inconsistent ingredients which are obtained from animals, including humans, the ratio of ingredients may vary whenever they are produced, thus causing various problems not only for experimental application but also for industrial application. Therefore, it is desirable to minimize the use of serum or use a serum-free medium. Therefore, for clinical applications of cryopreserved cells, the use of specific cryoprotective agents containing approved substances and non-animal biological materials is necessary.

In addition, dehydration, formation of oxygen free radicals, and apoptosis are major causes of decreased cell viability during or after freezing. Disaccharides such as sucrose and trehalose have been widely used as non-toxic natural cryoprotective agents, as freeze-drying additives, and as cell membrane stabilizers during dehydration. Peroxide radicals cause oxidative damage to cells, including lipid peroxidation, protein oxidation, and DNA damage.

Accordingly, as a result of extensive efforts to develop a cryopreservation formulation for cells such as stem cells that can minimize the decrease in cell viability due to peroxide radicals during cryopreservation of cells, preferably stem cells, the present inventors found that the cell viability after thawing was significantly increased when cells such as stem cells were cryopreserved using a cryopreservation formulation containing glutathione. Based on this finding, the present invention has been completed.

### [Disclosure]

Therefore, the present invention has been made in view of the above problems and it is one object of the present invention to provide a cryopreservation formulation for cells, preferably stem cells, that can significantly improve cell viability upon thawing after cryopreservation.

In accordance with one aspect of the present invention, the above and other objects can be accomplished by the provision of a cryopreservation formulation for cells, preferably a cryopreservation formulation for stem cells, containing glutathione and stem cells.

### [Description of Drawings]

FIG. 1 shows the results of screening antioxidants for stem cell cryopreservation formulations.
FIG. 2 shows the results of optimization of glutathione concentration for stem cell cryopreservation formulations.
FIG. 3 shows the results of measuring cell viability after freezing stem cells at a low concentration (5 x 10⁵ cells/500 *µ*ℓ) for one week using the cryopreservation formulation (4) according to the present invention, followed by thawing and culturing for 4 and 8 hours.
FIG. 4 shows the results of measuring cell viability after freezing stem cells at a high concentration (5 x 10⁶ cells/100 *µ*ℓ) for one week using the cryopreservation formulation (4) according to the present invention, followed by thawing and culturing for 4 and 8 hours.
FIG. 5 shows the results of measurement of cell viability after freezing stem cells for 1 month using a cryopreservation formulation (EN001 suspending agent) according to the present invention, followed by thawing and culturing for 4 and 8 hours.
FIG. 6 shows the results of observation of cell aggregation (A) and cell viability over time after freezing stem cells for 18 months using the cryopreservation formulation (EN001 suspending agent) according to the present invention, followed by thawing.
FIG. 7 shows the results of measurement of the stability of umbilical cord-derived mesenchymal stem cells (WJ-MSC), umbilical cord blood-derived mesenchymal stem cells (UCB-MSC), adipose-derived mesenchymal stem cells (AD-MSC), placental-derived mesenchymal stem cells (PL-MSC), fibroblasts, and the brain cancer cell line H4 after freezing of the cryopreservation formulation according to the present invention, followed by thawing at 4°C and 37°C, respectively.

### [Best Mode]

Unless defined otherwise, all technical and scientific terms used herein have the same meanings as understood by those skilled in the art to which the present invention pertains. In general, the nomenclature used herein is well-known in the art and is commonly used.

According to the present invention, to develop a cryopreservation formulation for cells such as stem cells that minimizes the loss of cell viability caused by peroxide radicals during cryopreservation of cells, preferably stem cells, the stem cells were frozen using various antioxidants and then thawed, and cell viability was determined. The results showed that, among these antioxidants, when the stem cells were cryopreserved using a cryopreservation formulation containing glutathione, cell viability significantly increased after thawing.

Therefore, the present invention relates to a formulation for cell cryopreservation containing glutathione and cells, preferably stem cells.

The formulation for cell cryopreservation according to the present invention may be prepared using Hartmann solution as a base solution and further comprising human serum albumin and DMSO.

In the present invention, the glutathione may be present at a concentration of 50 to 1,000 µM, preferably 50 to 500 µM, more preferably 50 to 200 µM, and even more preferably 60 to 150 µM.

In the present invention, the human serum albumin may be present at a concentration of 1 to 30%.

In the present invention, the DMSO may be present at a concentration of 0.5 to 30%.

In the present invention, the cells, preferably stem cells, may be present at a concentration of 5 x 10⁵ to 1 x 10⁷ cells/mL.

In the present invention, the Hartmann solution may contain 0.1 to 0.5 g/L of calcium chloride, 0.1 to 0.8 g/L of potassium chloride, 2 to 10 g/L of sodium chloride, and 1 to 5 g/L of sodium lactate, and preferably contains 0.27 g/L of calcium chloride, 0.4 g/L of potassium chloride, 6 g/L of sodium chloride, and 3.22 g/L of sodium lactate.

### [Example]

Hereinafter, the present invention will be described in more detail with reference to examples. However, it will be obvious in the art that various alterations and modifications are possible within the scope and technical idea of the present invention, and that such alterations and modifications fall within the scope of the appended claims.

### Example 1: Screening of antioxidants for stem cell cryopreservation formulation

In this example, a cryopreservation formulation for stem cells was established using umbilical cord blood-derived mesenchymal stem cells.

Umbilical cords and placentas were obtained by collaborative research with the Department of Obstetrics and Gynecology at Samsung Medical Center, Seoul, in accordance with the standards of the Samsung Medical Center's IRB (IRB# 2015-09-023-003) and then umbilical cord-derived mesenchymal stem cells (WJ-MSCs) were isolated from the umbilical cords according to the method of PARK et al. (Arch. Pharm. Res. 39: 1171-1179, 2016). Specifically, the umbilical cord was cut into 3-4 cm lengths, the tissue was finely chopped and treated with collagenase solution (Gibco, USA) for 60 to 90 minutes to degrade the extracellular matrix, and 0.25% trypsin (Gibco, USA) was added to further induce digestion for 30 minutes at 37°C. Then, fetal bovine serum (FBS; Biowest, USA) was added and cells were obtained by centrifugation at 1,000 g for 10 minutes, and then were cultured in a 5% CO₂ environment at 37°C using MEM Alpha (Minimum Essential Medium, Invitrogen-Gibco, Rockville, MD) medium containing 10% FBS (fetal bovine serum, Invitrogen-Gibco) and 50 *µ*g/mL gentamicin (Invitrogen-Gibco) to obtain human umbilical cord-derived mesenchymal stem cells (WJ-MSCs), which were then used in the experiment.

Typical stem cell cryopreservation formulations are prepared using Ringer's solution as a base solution, DMSO as a cryoprotective agent, and human serum albumin (Table 1).

**[Table 1]**

| Composition of typical cryopreservation formulation | | |
|---|---|---|
| | Ingredient | Function |
| 1 | Ringer's solution | Base solution |
| 2 | DMSO | Cryopreservation |
| 3 | Human serum albumin (HSA) | Cryopreservation and cell recovery (FBS alternative) |
| 4 | Anti-oxidant | Cell recovery |

In this example, in order to select an antioxidant that can minimize the decrease in cell viability due to peroxide radicals during cryopreservation of stem cells using an antioxidant, acetadote, fursultiamine hydrochloride, vitamin C, and glutathione were used as antioxidants. The WJ-MSCs were seeded at 1x10⁴ cells in a 96-well plate and cultured for 24 hours. The effects of the antioxidants on the cell viability of WJ-MSCs were determined by a CCK-8 assay.

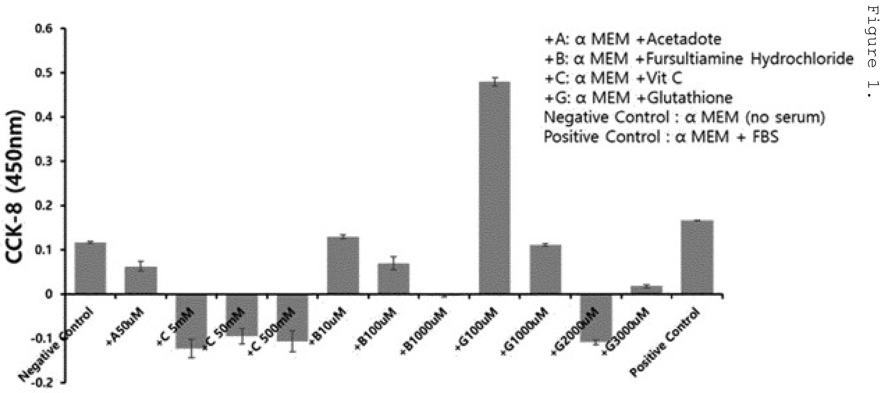

As shown in FIG. 1, the result showed that, when glutathione was used as an antioxidant, cell viability was the highest and cell proliferation was further increased.

### Example 2: Determination of the optimal concentration of glutathione in cryopreservation formulation

In order to determine the optimal concentration of glutathione in the cryopreservation formulation, the WJ-MSCs were treated with hydrogen peroxide (H₂O₂) at concentrations of 0 µM, 10 µM, 50 µM, and 100 µM, to induce stress caused by reactive oxygen species, and then further treated with glutathione at concentrations of 0 µM, 10 µM, 50 µM, and 100 µM, and cell recovery was evaluated using a CCK-8 assay.

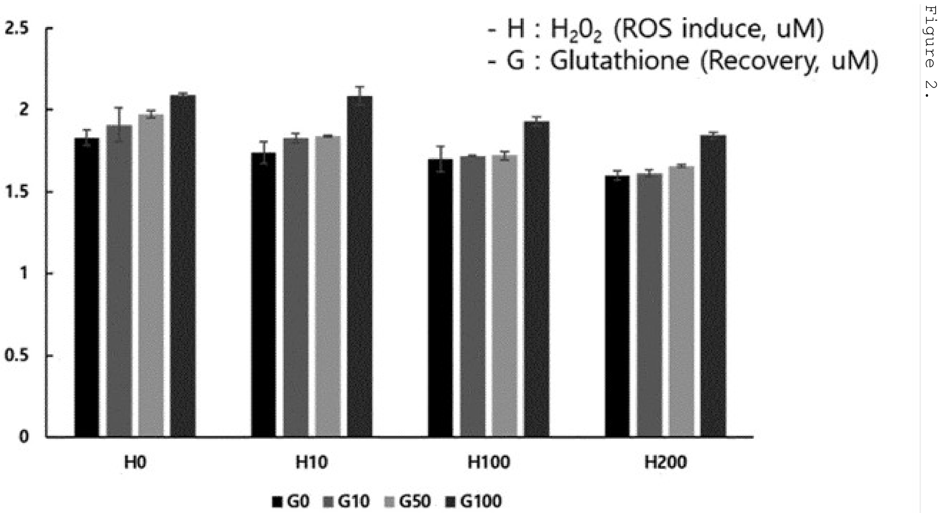

As shown in FIG. 2, the results showed that the highest cell viability was observed when glutathione was used at 100 µM, and it was determined to use 100 µM glutathione for the cryopreservation formulation.

### Example 3: Determination of the viability of stem cell cryopreservation formulations

To identify the optimal Ringer's solution suitable for stem cell cryopreservation formulations, the short-term stability was evaluated when commercially available cryopreservation formulations CS5 (Sigma, USA) and CS10 (Sigma, USA) were used as controls, and DSL 1:2:3 solution (CJ Healthcare) and Hartmann solution (Innoen) were used as base solutions.

The WJ-MSCs cryopreserved for one week at a low concentration (5 x 10⁵ cells/500 *µ*ℓ) were thawed at 4°C and 37°C, and then allowed to stand for 4 and 8 hours. Cell viability was measured.

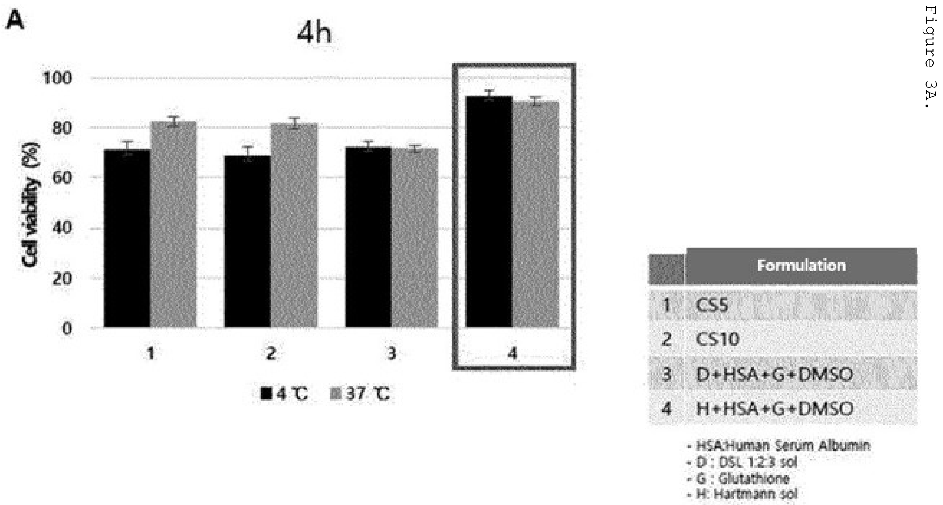

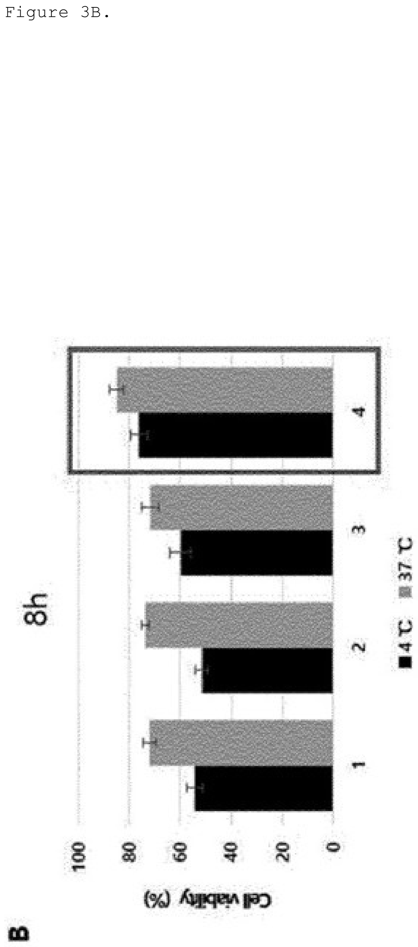

As shown in FIG. 3, the highest cell viability was obtained when the combination of H+HSA+G+DMSO (Hartmann solution + human serum albumin + glutathione + DMSO) was used as the cryopreservation formulation.

Furthermore, the WJ-MSCs cryopreserved for one week at a high concentration (5 x 10⁶ cells/100 µl) were thawed at 4°C and 37°C, and then allowed to stand for 4 and 8 hours. Cell viability was measured.

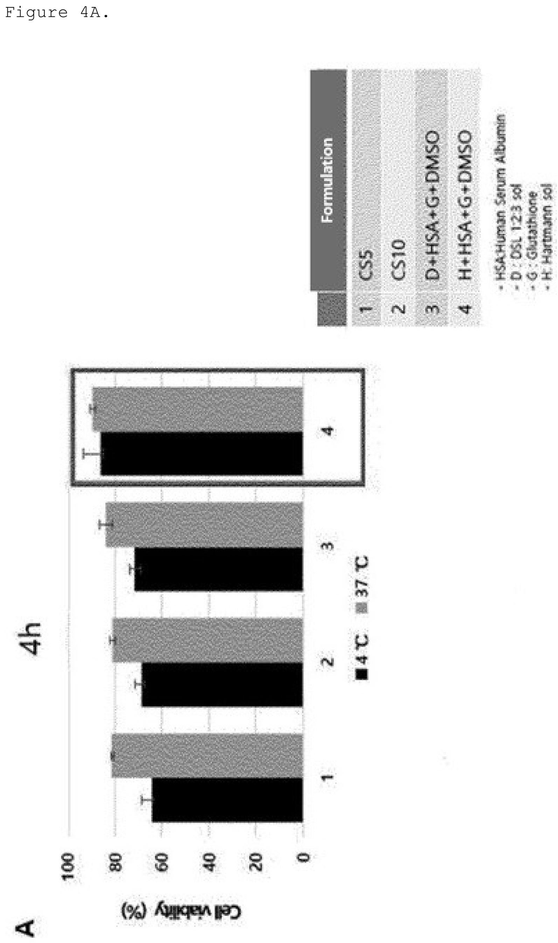

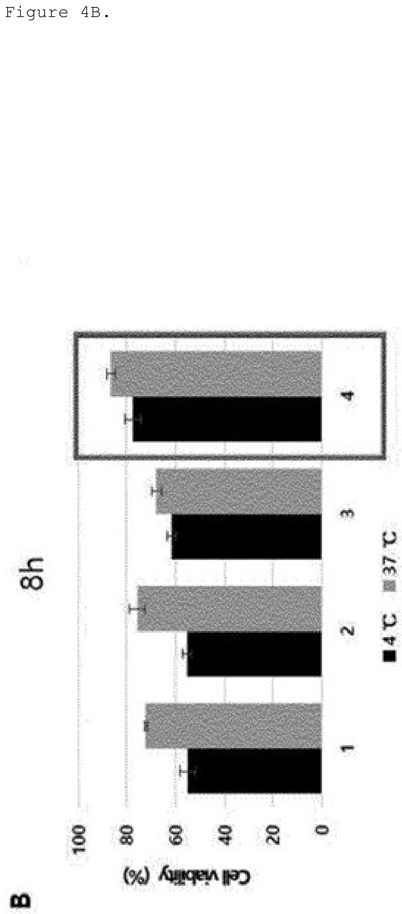

As shown in FIG. 4, the result showed that, when using high-concentration stem cells, the cryopreservation formulation containing H+HSA+G+DMSO exhibited the highest cell viability.

Therefore, the cell (preferably, stem cell) cryopreservation formulation according to the present invention was determined to be a formulation containing human serum albumin (HSA), glutathione, and DMSO in Hartmann solution (Innoen), and was designated "EN001 suspending agent".

### Example 4: Determination of the stability of stem cell cryopreservation formulations

Short-term stability was evaluated using commercially available cryopreservation formulations, CS5 (Sigma, USA) and CS10 (Sigma, USA), and a cryopreservation formulation containing FBS and DMSO, in comparison with "EN001 suspending agent", which is a stem cell cryopreservation formulation of the present invention.

The cells were cryopreserved in each cryopreservation formulation for one month and then were thawed at 4°C and 37°C. Cell viability was measured after 4 and 8 hours.

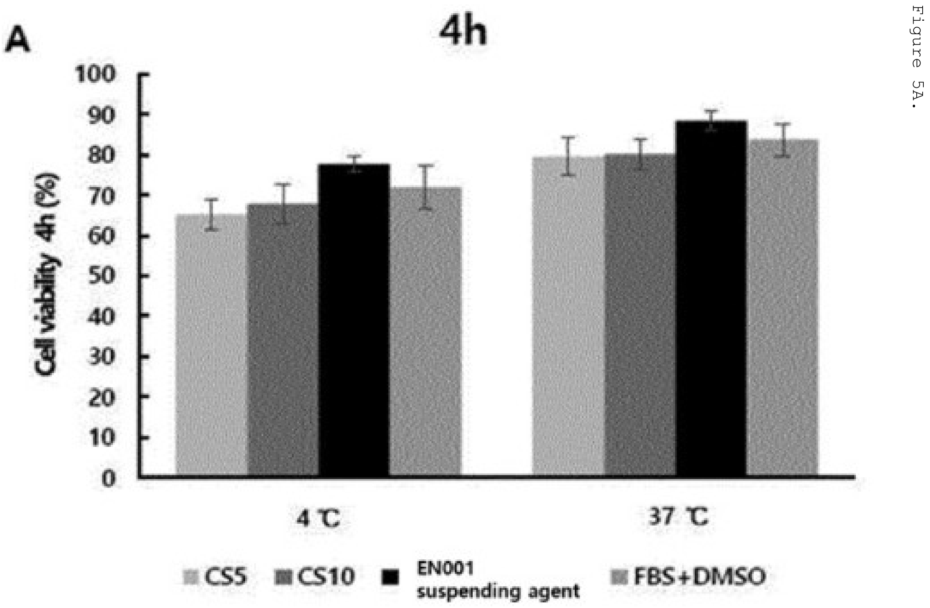

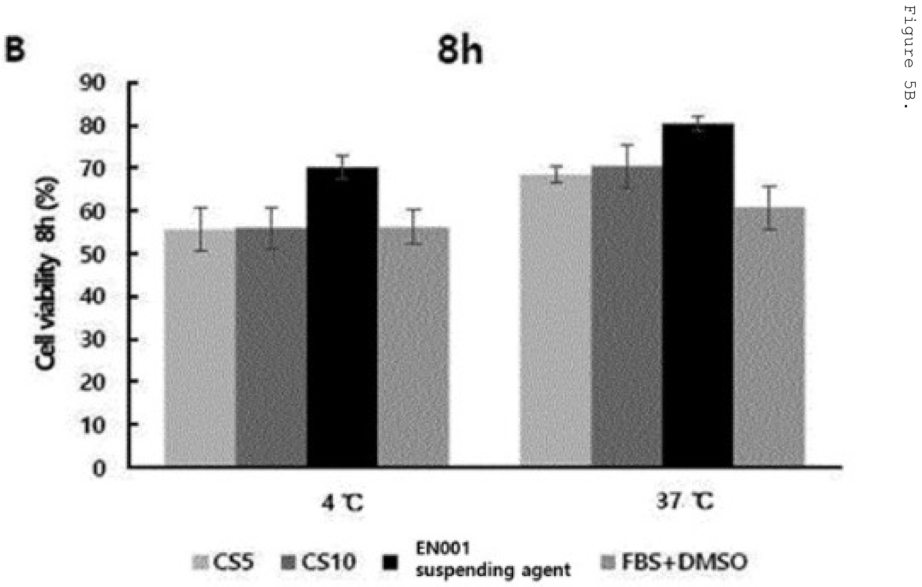

As shown in FIG. 5, the result showed that the cryopreservation formulation of the present invention exhibited the highest cell viability.

Furthermore, the cells were cryopreserved in the "EN001 suspending agent", which is a stem cell cryopreservation formulation of the present invention for 18 months, and were thawed, and the ratio of single cells to cell clumps, and the cell viability were measured.

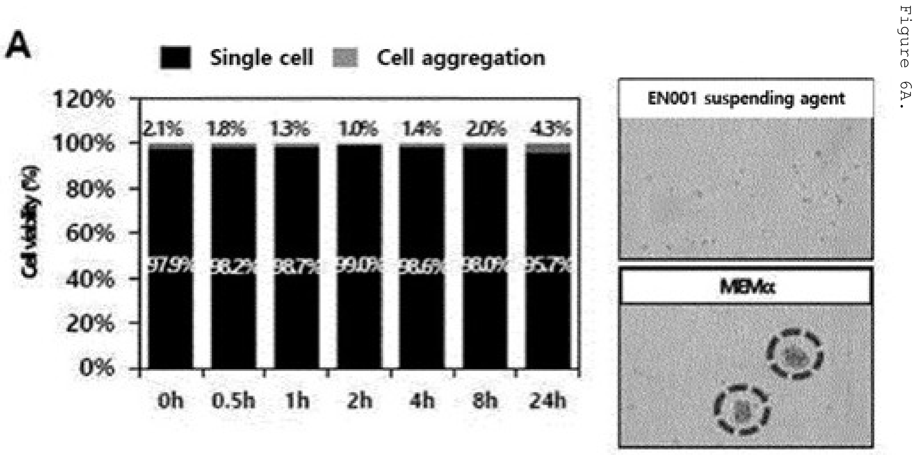

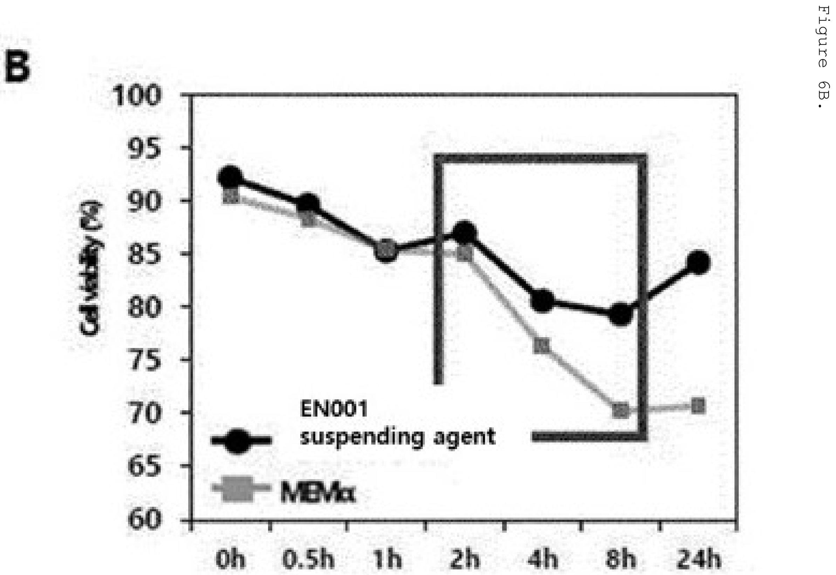

As shown in FIG. 6, "EN001 suspending agent", which is a stem cell cryopreservation formulation of the present invention, exhibited a significantly lower cell clumping rate (A), and sustained cell viability (B) of 80% or higher.

### Example 5: Determination of expanded applicability of stem cell cryopreservation formulation

Umbilical cord blood-derived mesenchymal stem cells (UCB-MSCs), adipose-derived mesenchymal stem cells (AD-MSCs), placental-derived mesenchymal stem cells (PL-MSCs), fibroblasts, and the brain cancer cell line H4 as well as umbilical cord-derived mesenchymal stem cells (WJ-MSCs) were cryopreserved using the cryopreservation formulation according to the present invention for one month and were then thawed at 4°C and 37°C, and the stability of the cells was determined.

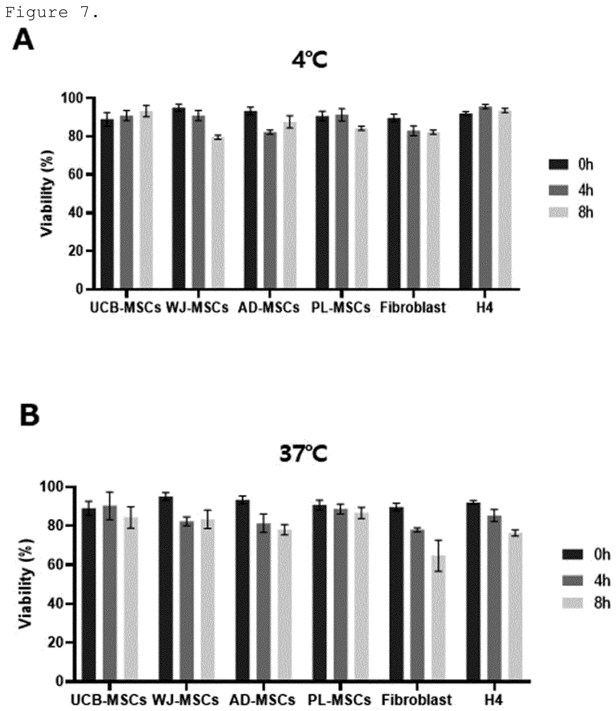

As shown in FIG. 7, all cell types used exhibited high cell viability when thawed at 4°C.

### [Industrial Applicability]

According to the present invention, cell viability can be improved upon thawing after freezing of stem cells, thereby preventing a decrease in therapeutic efficacy that may occur during the storage and transportation of cell therapeutics such as stem cells.

Although the preferred embodiments of the present invention have been disclosed for illustrative purposes, those skilled in the art will appreciate that various modifications, additions and substitutions are possible, without departing from the scope and spirit of the invention as disclosed in the accompanying claims.

## Claims

1. A formulation for cell cryopreservation comprising glutathione and cells.

2. The formulation for cell cryopreservation according to claim 1, further comprising Hartmann solution, human serum albumin, and DMSO.

3. The formulation for cell cryopreservation according to claim 1, wherein the glutathione is present at a concentration of 50 to 1,000 µM.

4. The formulation for cell cryopreservation according to claim 2, wherein the human serum albumin is present at a concentration of 1 to 30%.

5. The formulation for cell cryopreservation according to claim 2, wherein the DMSO is present at a concentration of 0.5 to 30%.

6. The formulation for cell cryopreservation according to claim 1, wherein the cells are stem cells.

7. The formulation for cell cryopreservation according to claim 6, wherein the stem cells are present at a concentration of 5x10⁵ to 1x10⁷ cells/mL.

8. The formulation for cell cryopreservation according to claim 2, wherein the Hartmann solution comprises 0.1 to 0.5 g/L of calcium chloride, 0.1 to 0.8 g/L of potassium chloride, 2 to 10 g/L of sodium chloride, and 1 to 5 g/L of sodium lactate.
